# EUROPEAN PATENT SPECIFICATION

(11) **EP 0 698 382 B1**
(45) Date of publication and mention of the grant of the patent: **05.04.2000**
(21) Application number: 95305766.8
(22) Date of filing: 18.08.1995
(51) Int. Cl.: A61F 2/30, A61L 27/00, B29C 45/03, A61F 2/34, A61F 2/38

(54) **Prosthetic bearing element and process for making such an element**
Prothesenlagerelement und Verfahren zur Herstellung eines solchen Elements
Elément de palier prothétique et procédé pour fabriquer un tel élément

(30) Priority: 25.08.1994 GB 9417288
(43) Date of publication of application: 28.02.1996
(73) Proprietor: HOWMEDICA INTERNATIONAL INC., Shannon Co. Clare (IE)
(72) Inventor: Smith, Nigel, Dr., Wokingham RG11 1LH (GB); Doyle, Christina, Dr., Cranleigh, Surrey GU6 8PJ (GB); Jones, Eric, Dr., County Limerick (IE)
(74) Representative: Bridge-Butler, Alan James

(56) References cited:
- EP-A- 0 203 719
- EP-A- 0 297 789
- EP-A- 0 351 545
- EP-A- 0 461 019
- EP-A- 0 495 341
- EP-A- 0 497 079
- WO-A-90/11060
- DE-A- 2 247 721
- FR-A- 2 695 063
- GB-A- 2 126 096
- US-A- 5 047 054
- US-A- 5 201 881

## Description

This invention relates to a prosthetic bearing element and to a process for making such an element.

A prosthetic bearing element as defined in the preamble of claim 1 is disclosed in EP-A-0 297 789.

At the present time wear of the well known polyethylene acetabular components in hips, the tibial component in knees, and other prosthetic bearing inserts limits the life of these artificial joints. Normally orthopaedic devices for joint construction and reconstruction comprise polyethylene bearings on metal or ceramic or metal on metal for articulation. The polyethylene bearing is often held in a metal shell, for example in hip constructions or a tray in knee constructions. Conveniently bearing liners made from ultra-high molecular weight polyethylene are fixed via a snap fit into the metal backing.

The possible use of elastomers in bearings to provide fluid film lubrication at low velocity, and with a low viscosity lubricant such as in a reconstructed joint has been published in laboratory studies which show that such "soft" bearings provide lower coefficient of friction compared with standard polyethylene versus metal bearings. Such a material is polyurethane.

Because of the fluid film lubrication, it is far less likely that the two bearing surfaces touch during use and thus wear is lower.

It is however difficult to apply this concept for a number of reasons. One difficulty lies in selecting a polyurethane or other elastomer which will not be degraded by the human body so great care must be taken in selecting the polyurethane material used. As mentioned above polyethylene bearing are usually held by physical means in, for example, a metal shell but it is not possible to use this approach with polyurethane because as its stiffness is so much lower than the metal it would be possible for it to extrude from the shell or be easily moved relative to the shell causing damage. Also lower stiffness would lead to greater interface strain.

Because of the above difficulties the Applicants have tried several different approaches. Initially, for ease of use and conventional appearance, it was attempted to bond a polyurethane liner onto a polyethylene backing, similar to pre-existing polyethylene components. This assembly could then be held in a metal shell or cemented depending on the surgeon's preference and clinical judgement.

The present invention therefore is related to a prosthetic bearing element which uses an elastomeric polyurethane bearing material and to a method of manufacturing such a bearing element which overcomes some of the difficulties referred to above.

According to the present invention a prosthetic bearing element comprises a backing which supports a bearing liner having a bearing surface, said backing being made from a "rigid" polymeric material having characterised in that said "rigid" polymeric material has a minimum hardness value of 65 N/mm² and said bearing liner is made from a "soft" elastomeric polyurethane material having a hardness value of 3.0 to 9.0 N/mm², using hardness testing method BS 2782; Pt3 method 365D.

It has been found that bearing elements of this type can be made by a moulding process with the surprising result that when the elastomeric material is applied onto a "rigid" polyurethane backing the stability of the interface appears to be better than that predicted by simple mechanical fit. It is not clear whether there is some form of chemical bond and what precisely causes the excellent adhesion between the two materials. It is possible that an interpenetrating network of the "soft" elastomeric polyurethane into the rigid polymer results.

Preferably therefore the liner is bonded to the backing and the backing itself can also be made from a range of rigid polyurethanes. Suitable polyurethane formulations include those based upon macrogycols formed from polyether glycols i.e. polytetramethylene ether glycol, or polyhexamethylene ether glycol, or polyoctamethylene ether glycol or polydecamethylene ether glycol, or polyester based glycols i.e. poly hexamethylene adipate glycol. The aforementioned macroglycols which are suited to reaction with suitable diisocyanates i.e. 4,4'-methylene bisphenyl diisocyanate, or 4,4'-methylene biscyclohexane diisocyanate, or 2,4-toluene diisocyanate and reacted further with chain extenders i.e. 1,4-butanediol, 1,6-hexanediol, ethylene diamine, 1,4-cyclohexane diamine. Polyurethane of different compositions can be synthesised by those skilled in the art from a range of these materials in many combinations.

A suitable material is Corothane 75D, a rigid segmented linear polyurethane polymer comprising of a polycarbonate based macroglycol, poly(1,6-hexyl 1,2-ethyl carbonate)diol reacted with 4,4'-methylene bisphenyl diisocyanate and chain extended using 1,4-butanediol. The hardness being determined by the ratio of hard segments (formed by the isocyanate and chain extended domains) to the soft segments (formed by the macrogycol portion of the polymer chain)(Corothane is a Registered Trade Mark of Corvita Corporation) or polymethylmethacrylate, or carbon fibre reinforced polybutyleneterphthlate (CFR-PBT). Another alternative material which can be used is carbon fibre reinforced polyetheretherketone (CFR-PEEK).

In a preferred construction the bearing liner is made from Corothane 80A.

A soft segmented linear polyurethane polymer comprising of a polycarbonate based macroglycol, poly(1,6-hexyl 1,2-ethyl carbonate)diol reacted with 4,4'-methylene bisphenyl diisocyanate and chain extended using 1,4-butanediol.

The invention can be applied to an acetabular cup or any other suitable prosthetic bearing.

If desired two or more bearing liners can be provided on the backing thus, the bearing element can be used in this manner in the form of a meniscal or conventional bearing for use in a knee prosthesis.

The invention also includes a prosthetic implant incorporating a bearing element as set forth above.

A process, according to the invention, for making a prosthetic bearing element as set forth above can include a two stage moulding process in which the backing or liner is moulded first and the appropriate liner or backing is moulded onto it.

Thus, the two parts are moulded consecutively rather than simultaneously.

Investigative studies by scanning electron microscopy (SEM), dynamic contact angle (DCA) and fourier transform infra-red spectroscopy (FTIR) have been undertaking to examine the interfacial region to determine the structural changes that occur.

The introduction of barium sulphate (BaSO₄) at 5% m/m into the elastomer, Corothane 80A, have shown the interface zone to be discrete at 2 - 5 micron and reproducible. In addition the structural composition of the Corothane 80A and Corothane 75D polyetherurethanes has been examined by recording their spectra over a series of sectors (5µM X 220µM) across a section through the interface region.

This FTIR technique allows the ratio of hard to soft segments in the linear polyetherurethanes to be quantified. The results show that the structure has been modified in the Corothane 75D to gradually change the composition from the interface zone to approximately 50 microns into the bulk as a result of the second moulding operation using Corothane 80A. It is this structural modification that occurs via the overmoulding of the rigid shell materials (Corothane 75D) with the elastomer (Corothane 80A) that appears to provide the adhesion between the two linear polyetherurethanes.

The invention can be performed in various ways and some embodiments together with details of the moulding process will now be described by way of example and with reference to the accompanying drawings in which :
Figure 1 is a cross-sectional view through a typical acetabular cup incorporating the invention;
Figure 2 is a cross-sectional view through a meniscal bearing for use in a knee prosthesis incorporating the invention;
Figure 3 diagrammatically illustrates three stages of moulding a prosthetic bearing element in which the backing is moulded first in a two-part process;
Figure 4 is a diagrammatic illustration showing the stages of moulding a similar bearing element to that shown in Figure 1 moulding the bearing liner first in a two-part process;
Figure 5 is a cross-sectional view through an injection mould for use in the process according to the invention; and,
Figure 6 is a diagrammatic side elevation of an injection moulding machine showing various areas of the injection moulding barrel.

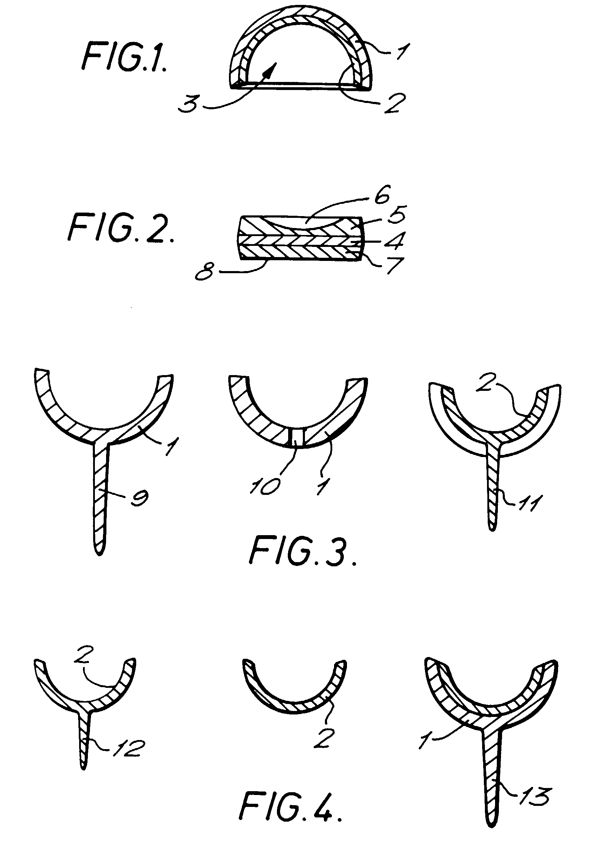

Figure 1 is a cross-section through a prosthetic hip cup according to the invention and which comprises an outer backing 1 which is made from a "rigid" polymeric material having a minimum hardness value of 65 N/mm². The backing 1 supports a bearing liner 2 made from a "soft" elastomeric polyurethane material having a hardness value of 3.0 to 9.0 N/mm² and a range thickness 1 to 5 mm.

The terms "rigid" and "soft" are used throughout the specification to indicate the relative properties of the material from which the backing and the liner are made.

The bearing surface of the cup shown in Figure 1 is indicated by reference numeral 3.

Figure 2 shows a meniscal bearing surface for use with the tray of a knee prosthesis. The bearing comprises a backing 4 made of a "rigid" polymeric material having a minimum hardness value of 65/Nmm² and an upper liner 5 having a bearing surface 6 and a lower liner 7 having a bearing surface 8. The liners 5 and 7 are made from a "soft" elastomeric polyurethane material having a hardness value of 3.0 to 9.0 N/mm² and a range of thickness 1 to 5 mm.

Figure 3 shows diagrammatically a process for moulding a bearing element of the kind shown in Figure 1. The backing 1 is moulded first in a suitable injection mould and which leaves a sprue 9. The backing is now taken out of the mould and the sprue 9 removed. A hole 10 is drilled through the backing 1 which is then replaced in the mould and the liner 2 is moulded by utilising the hole 10. The sprue left by the liner 2 is indicated by reference numeral 11. this is then removed to provide the completed bearing.

Figure 4 shows an alternative way of moulding the bearing element and in this arrangement the liner 2 is moulded first and there is a sprue 12. The liner 2 is taken out of the mould, the sprue 12 removed and the liner replaced in the mould where the backing 1 is moulded onto it. This again produces a sprue which is subsequently removed to provide the finished bearing element.

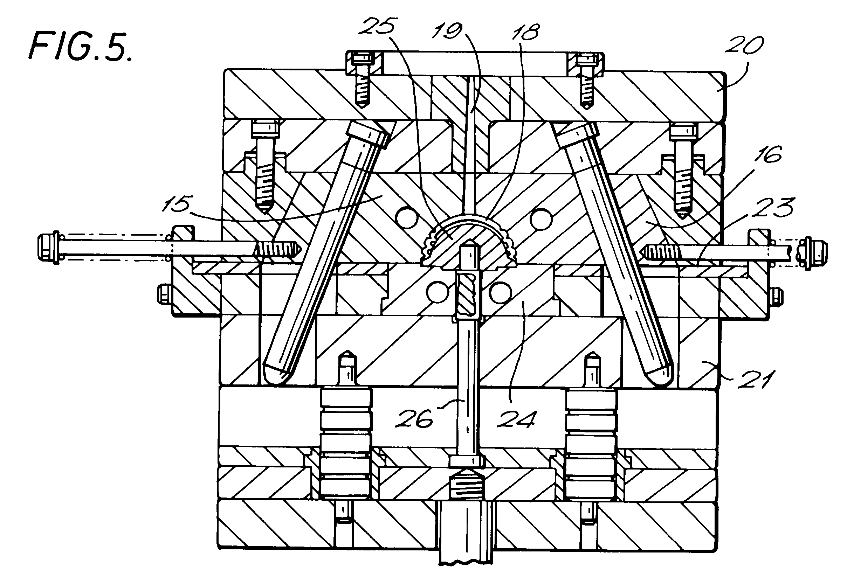

Figure 5 shows a suitable injection mould which can be used in the process. The general construction of the mould will be familiar to those skilled in the art and will not therefore be described in detail. The mould includes a pair of backing plates 15, 16 which together form a mould cavity 18 which can be fed from an injection moulding machine through an opening 19.

The mould is in two general parts, 20 and 21, and can be opened along a line 23.

The part of the mould carries a mounting 24 on which can be secured a male moulding element 25. This is secured by a bolt 26.

In order to mould bearing elements of different sizes a number of backing plates 15, 16 and mould elements 25 are provided.

In order to mould in the process shown in Figure 3, that is by moulding the backing element 1 first, a suitably dimensioned moulded element 25 is secured in place together with appropriate backing plates 15 and 16 and the mould press is operated to inject material through the opening 19 into the moulding space 18 to form a backing of the kind shown at the left hand side of figure 3. The mould is now opened along the line 23 and the moulded element 25 together with the backing 1 is detached. The backing, as moulded, is then removed from the mould element 25 and the sprue 9 which has formed in the opening 19 is removed. A hole 10 is now drilled in the backing and the backing is replaced in the mould with a different mould element 25 of smaller dimensions so that there is now a mould opening between the backing 1 and the surface of the mould element 25. Moulding material is now injected through the opening 19 and the hole 10 in the backing 1 so that it fills the space between the backing and the mould element 25. With moulding completed the mould is again split, the moulded bearing element separated from the moulding element 25 and the sprue 11 which has formed in the opening 19 is removed. A hole 10 is now drilled in the backing and the backing is replaced in the mould with a different mould element 25 of smaller dimensions so that there is now a mould opening between the backing 1 and the surface of the mould element 25. Moulding material is now injected through the opening 19 and the hole 10 in thebacking so that it fills the space between the backing and the mould element 25. With moulding completed the mould is again split, the moulded bearing element separated from the moulding element 25 and the sprue 11 which has formed in the opening 19 is removed.

In order to mould according to the process shown in Figure 4 with the same apparatus the process is carried out by using appropriately sized backing plates 15 and 16 and mould element 25 to produce the inner lining. With the sprue 12 removed the lining is replaced in the mould but this time with different backing plates 15, 16 which provide a cavity between the liner 2 and the edges of the cavity 18. The backing is now moulded in place, is removed in a manner described above and the sprue 13 is removed.

It will be appreciated that the above is a relatively simple way of moulding according to the process and other moulding techniques can be employed.

Using the above type of apparatus the following combinations of materials have been moulded using the process set forth and have produced satisfactory examples.

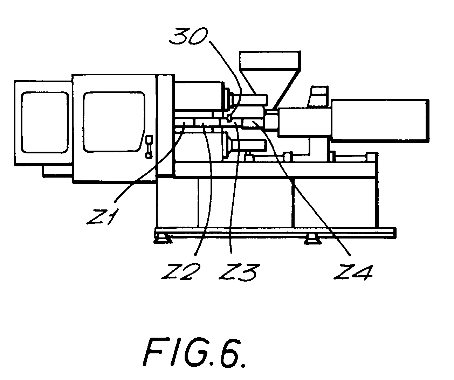

Figure 6 shows a typical commercial injection moulding machine and in which the injection moulding barrel is indicated by reference numeral 30. The various areas along the extending length of the barrel 30 are indicated by reference numerals Z1, Z2, Z3 and Z4. Reference to the temperatures in the barrel are shown in the following examples.

The screw speed and injection speed referred to are dimensionless covering an analogue range from 1.0 to 5.0. They are varied from time to time dependent upon cavity volume or melt viscosity.
1. Moulding CSIRO 85A (lot no 1267-1193)onto Corothane C75D

| Injection conditions | | |
|---|---|---|
| Times: | Inj (15 s); Holding (20 s) | |
| Pressure: | Injection (50 bar), Hold (25 bar) | |
| Cooling mould: | 10°C (20 s) | |
| Screw speed: | 3.0 | |
| Injection speed: | 4.0 | |
| Injection | temp 205°C | (Z1) |
| barrel | 201°C | (Z2) |
| barrel | 201°C | (Z3) |
| barrel | 191°C | (Z4) |

Peel test samples No. 40 to 45 using ptfe tape as crack initiator.
2. Moulding Tecothane 93A onto Corothane C75D

| Injection conditions | | |
|---|---|---|
| Times: | Inj (15 s); Holding (20 s); cooling (20s) | |
| Pressure: | Injection (050 bar), Hold (25 bar) | |
| Cooling mould: | 10°C | |
| Screw speed: | 3.0 | |
| Injection speed: | 4.0 | |
| Injection | temp 205°C | (Z1) |
| barrel | 201°C | (Z2) |
| barrel | 200°C | (Z3) |
| barrel | 191°C | (Z4) |

Peel test samples No. 56 to 70 using ptfe tape as crack initiator.
3. Moulding Choroflex AL80A (lot no CF 137) onto Corothane C75D

| Injection conditions | | |
|---|---|---|
| Times: | Inj (15 s); Holding (20 s) | |
| Pressure: | Injection (63 bar), Hold (30 bar) | |
| Cooling mould: | 10°C (20 s) | |
| Screw speed: | 3.0 | |
| Injection speed: | 5.0 | |
| Injection | temp 205°C | (Z1) |
| barrel | 201°C | (Z2) |
| barrel | 201°C | (Z3) |
| barrel | 191°C | (Z4) |

Peel test samples No. 71 to 87 using ptfe tape as crack indicators.
4. Moulding Pellethane 80A (lot no. 2363) onto Corothane C75D

| Injection conditions | | |
|---|---|---|
| Times: | Inj (15 s); Holding (20 s) | |
| Pressure: | Injection (50 bar), Hold (25 bar) | |
| Cooling mould: | 10°C (20 s) | |
| Screw speed: | 3.0 | |
| Injection speed: | 5.0 | |
| Injection | temp 205°C | (Z1) |
| barrel | 201°C | (Z2) |
| barrel | 191°C | (Z3) |
| barrel | 160°C | (Z4) |

Peel test samples No. 88 to 102 using ptfe tape as crack indicator.
5. Moulding Corothane 55D (lot no PEA0072) onto Corothane C75D

| Injection conditions | | |
|---|---|---|
| Times: | Inj (15 s); Holding (20 s) | |
| Pressure: | Injection (50 bar), Hold (25 bar) | |
| Cooling mould: | 10°C (20 s) | |
| Screw speed: | 3.0 | |
| Injection speed: | 5.0 | |
| Injection | temp 222°C | (Z1) |
| barrel | 218°C | (Z2) |
| barrel | 209°C | (Z3) |
| barrel | 190°C | (Z4) |

Peel test samples No. 103 to 116 using ptfe tape as crack indicator.
6. Moulding Corothane 80A (lot no PEA00038) onto Tecothane C75D.

| Injection conditions | | |
|---|---|---|
| Times: | Inj (15 s); Holding (20 s) | |
| Pressure: | Injection (50 bar), Hold (25 bar) | |
| Cooling mould: | 10°C (20 s) | |
| Screw speed: | 3.0 | |
| Injection speed: | 5.0 | |
| Injection | temp 216°C | (Z1) |
| barrel | 216°C | (Z2) |
| barrel | 205°C | (Z3) |
| barrel | 160°C | (Z4) |

Peel test samples No. 117 to 141 using ptfe tape as crack indicator.

Rigid backing pieces were moulded from Corothane 75D as follows.

| Injection conditions | | |
|---|---|---|
| Times: | Inj (15 s); Holding (20 s) | |
| Pressure: | Injection (50 bar), Hold (25 bar) | |
| Cooling mould: | Ambiient (20 s) | |
| Screw speed: | 3.0 | |
| Injection speed: | 5.0 | |
| Injection | temp 229°C | (Z1) |
| barrel | 223°C | (Z2) |
| barrel | 211°C | (Z3) |
| | 200°C | (Z4) |

Such backing pieces were ready for use with suitable liners.

Liners themselves were also made individually from Corothane 80A under the following conditions.

| Injection conditions | | |
|---|---|---|
| Times: | Inj (15 s); Holding (20 s) | |
| Pressure: | Injection (59 bar), Hold (25 bar) | |
| Cooling mould: | 10°C (20 s) | |
| Screw speed: | 3.0 | |
| Injection speed: | 5.0 | |
| Injection | temp 208°C | (Z1) |
| barrel | 203°C | (Z2) |
| barrel | 191°C | (Z3) |
| barrel | 160°C | (Z4) |

These liners were suitable for use with backing of suitable material.

Further "rigid" backing materials can be used for example what are usually referred to as Composites, that is a synthetic resin material with, for example, a fibre reinforcement. Example A set out below was the use of carbon fibre reinforced polybutyleneterphthlate (CFR-PBT).

| | |
|---|---|
| Times | Inj (4s); Holding (4s) |
| Pressure | Injection (40 bar), Hold (10 bar) |
| Temp | Injection 240°C |
| | Mould 80°C |
| Injection speed | 3 |

Another alternative material which was successfully injected was polyetheretherketone (CFR-PEEK) as follows.

| | |
|---|---|
| Times | Inj (4s); Holding (4s) |
| Pressure | Injection (40 bar), Hold (12 bar) |
| Temp | Injection 390°C |
| | Mould 180°C |
| injection speed | 3 |

These materials were successfully used as backings, again with, for example, Corothane 80A.

Experiments were carried out to establish the relative hardnesses of different materials and hardness testing of the various elastomers was undertaken using BS 2782; Pt 3 Method 365D (Test load 49N - Shore 80 - 93A; Test load 358N - Shore 75D).

The results of the hardness examination are shown below

| **Materials** | **Hardness (Nmn**^{**-3**}**)** |
|---|---|
| Corothane 80A | 5.380 (σ 0.146) |
| Tecoflex EG93A | 4.468 (σ 0.050) |
| Tecothane TTI080A | 5.191 (σ 0.041) |
| Chronoflex AL80A | 3.977 (σ 0.030) |
| CSIRO 85A | 7.894 (σ 0.147) |
| Corothane 55D | 65 |
| Corothane 75D | 89.40 (σ 2.31 ) |
| Tecothane 75D | 84.5 (σ 2.59 ) |
| σ denotes standard abbreviation | |

As will be seen the difference between "rigid" and "soft" are apparent.

A peel test was performed on replicate specimens to assess the bond between the rigid backing and the flexible "soft" layer. the test used is a standard test in which a 3mm thick flexible layer is bonded to an 8mm thick rigid backing with the last 35mm unbonded by masking the interface using PTFE tape. The flexible layer is gripped and pulled at a peel angle of 60° in a Lloyd R6000 Universal testing machine. The force required to peel the layers apart is recorded as the peeling continues for about 50mm.

The table below shows the results of this test where N is the mean peel force, the Strain Energy is the area under the force vs. peel distance curve and the adhesive Fracture Energy is the energy released per unit of new surface created on separating the interface.

| **Backing shell** | **Bearing elastomer** | **Peel force Force (N)** | **Strain energy (KJm**^{**-3**}**)** | **Fracture energy (JM**^{**-2**}**)** |
|---|---|---|---|---|
| UHMWPE | Corothane 80A | 10 | n.d. | n.d. |
| UHMWPE | Tecoflex | 1.3 | n.d. | n.d. |
| UHMWPE *pAA | Tecoflex | 2.9 | n.d. | n.d. |
| UHMWPE *pHEMA | Tecoflex | 2.9 | n.d. | n.d. |
| UHMWPE //NH₃ | Corothane 80A | 10 | 0.96 | 95 |
| UHMWPE //allylamine | Corothane 80A | 4.22 | 0.15 | 39.5 |
| e | | | | |
| Corothane 75D | Corothane 80A | 540 | 6,480 | 25,700 |
| Tecothane 75D | Corothane 80A | 523 | 9,760 | 23,520 |
| Corothane 75D | CSIRO 85A | 315 | n.d. | n.d. |
| Corothane 75D | Corothane 55D | 502 | n.d. | n.d. |
| Corothane 75D | Chronoflex AL80A | 403 | n.d. | n.d. |
| Corothane 75D | Pellethane 80A | 542 | n.d. | n.d. |
| n.d. denotes not determined | | | | |
| * denotes photochemical grafting | | | | |
| // denotes fuctionalisation of PE by plasma modification | | | | |
| NH₃ (ammonia plasma) | | | | |
| Allylamine (allyamine to quench radicals by an oxygen plasma) | | | | |
| PEI (polyethylenimine to quench radicals generated by an oxygenplasma) | | | | |

It will be seen that the peel force required for the first six combinations of materials is very low but immediately "rigid" and "soft" materials are applied together the peel force required rises dramatically.

This ability to resist separation is utilised in the preparation of the bearing elements of the kind set forth in the present invention.

## Claims

1. A prosthetic bearing element comprising a backing (1, 4) which supports a bearing liner (2, 5, 7) having a bearing surface, said backing being made from a "rigid polymeric material characterised in that said "rigid" polymeric material has a minimum hardness value of 65 N/mm² and said bearing liner (2, 5, 7) is made from a "soft" elastomeric polyurethane material having a hardness value of 3.0 to 9.0 N/mm², using hardness testing method BS 2782; Pt3 method 365D.

2. A prosthetic bearing element as claimed in claim 1 in which said liner is bonded to said backing.

3. A prosthetic bearing element as claimed in claim 2 in which said backing is made from polyurethane.

4. A prosthetic bearing element as claimed in claim 3 in which said polyurethane is Corothane 75D.

5. A prosthetic bearing element as claimed in claim 2 in which said backing is made from carbon fibre reinforced polybutyleneterphthlate (CFR-PBT).

6. A prosthetic bearing element as claimed in claim 2 in which said backing material is made from polyetheretherketone (CFR-PEEK).

7. A prosthetic bearing element as claimed in claim 2 in which said backing material is made from polymethylmethacrylate.

8. A prosthetic bearing element as claimed in any one of the proceeding claims in which the bearing liner is made from Corothane 80A.

9. A prosthetic bearing element as claimed in claims 1 to 8 which is in the form of an acetabular cup.

10. A prosthetic bearing element as claimed in any one of the preceding claims 1 to 8 in which two or more being liners are provided on said backing.

11. A prosthetic bearing element as claimed in claim 9 which is in the form of a bearing insert for use in a knee prosthesis

12. A prosthetic orthopaedic or maxillofacial implant incorporating a bearing element as set forth in any one of the preceding claims.

13. A process for making a prosthetic bearing element as claimed in claim 1 which includes a two stage moulding process in which the backing (1, 4) or liner (2, 5, 7) is moulded first and the appropriate liner (2, 5, 7) or backing (1, 4) is moulded onto it.

## Patentansprüche

1. Prothetisches Lagerelement, umfassend einen Träger (1,4), der eine Lagerauskleidung (2,5,7) mit einer Lageroberfläche trägt, wobei der Träger aus einem "starren" Polymermaterial hergestellt ist, dadurch gekennzeichnet, daß das "starre" Polymermaterial einen Mindesthärtewert von 65 N/mm² aufweist, und die Lagerauskleidung (2,5,7) aus einem "weichen" elastomeren Polyurethanmaterial hergestellt ist, das einen Härtewert von 3,0 bis 9,0 N/mm² aufweist, wenn das Härtebestimmungsverfahren BS 2782; Pt 3 Verfahren 365 D benutzt wird.

2. Prothetisches Lagerelement nach Anspruch 1, bei dem die Auskleidung mit dem Träger verbunden ist.

3. Prothetisches Lagerelement nach Anspruch 2, bei dem der Träger aus Polyurethan hergestellt ist.

4. Prothetisches Lagerelement nach Anspruch 3, bei dem das Polyurethan Corothan 75D ist.

5. Prothetisches Lagerelement nach Anspruch 2, bei dem der Träger aus kohlefaserverstärktem Polybutylenterephthalat (CFR-PBT) hergestellt ist.

6. Prothetisches Lagerelement nach Anspruch 2, bei dem das Trägermaterial aus Polyetheretherketon (CFR-PEEK) besteht.

7. Prothetisches Lagerelement nach Anspruch 2, bei dem das Trägermaterial aus Polymethylmethacrylat besteht.

8. Prothetisches Lagerelement nach einem der vorangehenden Ansprüche, bei dem die Lagerauskleidung aus Corothan 80A hergestellt ist.

9. Prothetisches Lagerelement nach den Ansprüchen 1 bis 8, das in Form einer Hüftgelenkpfanne vorliegt.

10. Prothetisches Lagerelement nach einem der vorangehenden Ansprüche 1 bis 8, bei dem zwei oder mehr Lagerauskleidungen auf dem Träger vorgesehen sind.

11. Prothetisches Lagerelement nach Anspruch 9, das in Form eines Lagereinsatzes zur Verwendung in einer Knieprothese vorliegt.

12. Prothetisches orthopädisches oder Gesichtsknochen-Implantat, das ein Lagerelement nach einem der vorangehenden Ansprüche einschließt.

13. Verfahren zur Herstellung eines prothetischen Lagerelements nach Anspruch 1, das einen zweistufigen Formprozess einschließt, bei dem zuerst der Träger (1,4) oder die Auskleidung (2,5,7) geformt wird, und die passende Auskleidung (2,5,7) oder der passende Träger (1,4) darauf aufgeformt wird.

## Revendications

1. Elément de portée prothétique comprenant un support (1, 4) qui supporte une chemise de portée (2, 5, 7) présentant une surface de portée, ledit support étant fait en un matériau polymère "rigide" (2, 5, 7), caractérisé par le fait que ledit matériau polymère "rigide" a une valeur de dureté minimale de 65 N/mm² et par le fait que ladite chemise de portée (2, 5, 7) est réalisée en un matériau en élastomère de polyuréthanne "mou" ayant une valeur de dureté comprise entre 3,0 et 9,0 N/mm², mesurée en utilisant un procédé de mesure de la dureté BS 2782 ; Pt3 procédé 365 D.

2. Elément de portée prothétique selon la revendication 1, dans lequel ladite chemise est fixée audit support.

3. Elément de portée prothétique selon la revendication 2, dans lequel ledit support est réalisé en polyuréthanne.

4. Elément de portée prothétique selon la revendication 3, dans lequel ledit polyuréthanne est du Corothane 75D.

5. Elément de portée prothétique selon la revendication 2, dans lequel ledit support est réalisé en polytéréphthlate de butylène renforcé par des fibres de carbone (CFR-PBT).

6. Elément de portée prothétique selon la revendication 2, dans lequel ledit matériau de support est réalisé en polyéther-éthercétone (CFR-PEEK).

7. Elément de portée prothétique selon la revendication 2, dans lequel ledit matériau de support est réalisé en polyméthacrylate de méthyle.

8. Elément de portée prothétique selon l'une quelconque des revendications précédentes, dans lequel la chemise de portée est réalisée en Corothane 80A.

9. Elément de portée prothétique selon les revendications 1 à 8, qui se présente sous la forme d'une coupelle acétabulaire.

10. Elément de portée prothétique selon l'une quelconque des revendications 1 à 8 précédentes, dans lequel deux ou plusieurs chemises de portée sont disposées sur ledit support.

11. Elément de portée prothétique selon la revendication 9, qui se présente sous la forme d'une pièce d'insertion de portée destinée à être utilisée dans une prothèse de genou.

12. Implant orthopédique ou maxillo-facial prothétique incorporant un élément de portée selon l'une quelconque des revendications précédentes.

13. Procédé pour réaliser un élément de portée prothétique selon la revendication 1 qui comprend un processus de moulage à deux étapes dans lequel le support (1, 4) ou la chemise (2, 5, 7) est moulé en premier et la chemise (2, 5, 7) ou le support (1, 4) approprié est moulée sur celui-ci.
